**Europäisches Patentamt**

**European Patent Office**

**Office européen des brevets**

(19)

(11) Numéro de publication: **0 147 335 B1**

(12) **FASCICULE DE BREVET EUROPEEN**

(45) Date de publication du fascicule du brevet:
**20.04.88**

(21) Numéro de dépôt: **84402753.2**

(22) Date de dépôt: **28.12.84**

(51) Int. Cl.⁴: **A 61 K 9/22,** A 61 K 9/26, A 61 K 47/00

(54) Matrice pharmaceutique inerte à base de polycaprolactone et procédé de préparation d'une forme galénique orale.

(30) Priorité: **02.01.84 FR 8400015**

(43) Date de publication de la demande:
**03.07.85 Bulletin 85/27**

(45) Mention de la délivrance du brevet:
**20.04.88 Bulletin 88/16**

(84) Etats contractants désignés:
**AT BE CH DE FR GB IT LI LU NL SE**

(56) Documents cités:
**FR - A - 1 490 855
FR - A - 2 363 251
FR - A - 2 377 431
US - A - 3 929 937
US - A - 4 148 871**

**CHEMICAL ABSTRACTS, vol. 92, no. 16, 1980, page 410,
no. 135249h, Columbus, Ohio, US; F. CARLI et al.:
"Effect of microstructure on liquid capillary penetration
into porous drug carriers"**

(73) Titulaire: **LABORATOIRES D'HYGIENE ET DE
DIETETIQUE L.H.D. Société Anonyme dite:, 38 avenue
Hoche, F-75008 Paris (FR)**

(72) Inventeur: **Grouiller, Hervé, 81 avenue Victor Hugo,
F-21 000 Dijon (FR)**
Inventeur: **Christ, Fabien, 35 rue Vannerie, F-21000 Dijon
(FR)**

(74) Mandataire: **Combe, André, CABINET BEAU DE
LOMENIE 55, rue d'Amsterdam, F-75008 Paris (FR)**

## Description

La présente invention a trait à une nouvelle matrice physiologiquement inerte pour administration d'un médicament par voie orale. Cette nouvelle matrice comprend comme moyen essentiel une substance polymère: la polycaprolactone (en abrégé ci-après «PCL»). L'invention concerne également le procédé de préparation de la forme galénique à dureté et porosité contrôlées comprenant cette matrice.

On sait que dans le domaine de la galénique on a utilisé des substances telles que les dérivés de cellulose, les copolymères acryliques et le PVC, pour réaliser des matrices physiologiquement inertes. Or, il se trouve que ces substances, qui conviennent pour granulation par voie humide, ne conviennent pas toutes pour granulation par voie sèche. Ainsi, parmi ces substances, seuls les dérivés de cellulose, et en particulier l'éthylcellulose, peuvent être granulés par voie sèche et comprimés, mais ils donnent des matrices qui présentent l'inconvénient de regonfler après compression, qui sont trop friables et dont la porosité ne peut pas être réglée en fonction du principe actif incorporé. De plus, l'éthylcellulose, les copolymères acryliques et le PVC présentent l'inconvénient de limiter la teneur en médicament à une valeur inférieure à 40% en poids par rapport au poids de la préparation médicamenteuse renfermant la matrice, le médicament et les autres adjuvants galéniques (notamment un agent lubrifiant et un agent d'écoulement).

On connaît par ailleurs, notamment des articles de C.G. PITT et al., J. Biomed. Mater. Res., 13, pages 497–507 (1979), d'une part, et J. Pharm. Sci., 68 (N° 12), pages 1534–1538 (1979), d'autre part, l'utilisation de films de PCL pour réaliser des implants subdermiques permettant une libération d'un médicament dans le temps [3 à 12 mois ou plus selon le premier article de C.G. PITT et al. (voir page 498, ligne 4); 2 à 14 mois (calcul théorique) ou 20 à 200 jours ou plus (mesures in vitro et in vivo) selon le second article de C.G. PITT et al. (voir tableau I et pages 1537–1538)].

Ces films utilisables pour implantation, qui peuvent (i) renfermer le médicament dans leur masse, (ii) être présentés sous forme de couches superposées constituant des stratifiés ou des sandwichs, ou (iii), après avoir été enroulés ou repliés sur eux mêmes et scellés, servir d'enveloppes ou poches souples (capsules ou sachets) contenant dans leur cavité interne le médicament, ne conviennent pas pour administration per os dès lors que le médicament ne peut être libéré au cours de la durée normale de transit gastro-intestinal, et, qu'un phénomène dit de «couche barrière aqueuse», souligné par PITT et al. peut se manifester limitant la libération.

Selon l'invention, on préconise une nouvelle solution technique au problème galénique de la granulation sèche, qui permet de pallier les inconvénients susvisés, notamment ceux des dérivés de cellulose, des copolymères acryliques et du PVC, qui offre l'avantage (i) de pouvoir réaliser notamment des comprimés ne regonflant pas après compression, non friables, plastiques (c'est-à-dire pouvant absorber les chocs sans casser) ayant une structure poreuse et cohérente, renfermant jusqu'à 80% en poids de médicament et (ii) de contrôler, sans dégradation du principe actif, la porosité et la dureté des préparations médicamenteuses renfermant le médicament, la PCL et les autres adjuvants en vue de régler le relargage dans le temps. Cette solution technique, qui met en œuvre des grains de PCL, se différencie en outre de la technique d'implantation au moyen de films de PCL: après administration, le ou les principes actifs sont libérés lors du transit gastro-intestinal par les interstices présents entre les grains de PCL, alors qu'ils diffusent à travers les pores des films de PCL avec une vitesse beaucoup plus faible.

Le but de l'invention est de proposer une nouvelle matrice pour administration orale d'un médicament sous forme de comprimés de porosité et dureté contrôlées.

Selon l'invention, par principe actif, on entend toute substance thérapeutiquement active et efficace; par médicament, ledit principe actif ou tout mélange d'au moins deux principes actifs; et, par préparation médicamenteuse, toute composition renfermant un médicament en association avec un excipient physiologiquement acceptable, c'est-à-dire dans le cas d'espèce la matrice inerte et les autres adjuvants tels que notamment l'agent lubrifiant et l'agent d'écoulement.

Par matrice inerte, on entend ici le fait que la matrice n'est pas digérée dans l'organisme et qu'elle ne présente pas d'interaction avec les principes actifs et les fluides de l'organisme.

La matrice inerte selon l'invention pour administration orale d'un médicament qui comprend de la polycaprolactone est caractérisée en ce que la polycaprolactone qu'elle renferme a un poids moléculaire moyen compris entre 2000 et 70 000 et est sous forme de grains ayant un diamètre moyen compris entre 50 et 500 μm et présente un degré de cristallinité d'au moins 75%.

La PCL selon l'invention est une poly (ε-caprolactone) de formule $\{(CH_2)_5-CO-O\}_n$ où n est un nombre sensiblement voisin (i) de 17 pour un poids moléculaire moyen de 2000 et (ii) de 615 pour un poids moléculaire moyen de 70 000. De façon avantageuse, le poids moléculaire moyen de la PCL sera compris entre 30 000 et 45 000 et le degré de cristallinité sera compris entre 80 et 95%.

Pour régler la porosité et la dureté de la matrice, on procède par compression, puis, le cas échéant, par un traitement choisi parmi (i) le traitement thermique à une température comprise entre 45 et 70 °C pendant une durée inférieure ou égale à 20 minutes et (ii) le traitement au moyen d'un champ UHF (ultra-hautes fréquences) d'environ 1 MHz à environ 25 GHz pendant une durée inférieure ou égale à 60 s et notamment comprise entre 10 et 60 s.

La compression permet de réaliser l'agglomération des grains de la matrice entre eux et/ou avec le médicament et les autres adjuvants, et, par suite, la cohésion du produit final, et, d'assurer une certaine porosité en fonction de la force de compression. Cette compression est avantageusement réalisée sous une pression supérieure ou égale à 100 daN/cm$^2$ (soit 10$^5$ pascals) et de préférence comprise entre 200 daN/cm$^2$ et 2000 daN/cm$^2$ (soit de $2 \times 10^5$ pascals à $2 \times 10^6$ pascals).

Le calibrage de la porosité peut être ajusté selon le traitement thermique ou le traitement UHF susvisés, ce dernier étant avantageusement destiné à l'obtention des préparations médicamenteuses comprenant un principe actif sensible à des températures supérieures à 70 °C. Le traitement thermique confère préférentiellement une dureté superficielle alors que le traitement UHF confère préférentiellement une dureté dans la masse de la matrice. Avec les traitements thermique ou UHF, on obtient une matrice dont la porosité est telle que le diamètre moyen des pores constitués par les interstices présents entre les grains de PCL est compris entre 100 µm et 0,002 µm pour une granulométrie de la PCL comprise entre 500 µm et 50 µm.

Le procédé de préparation d'une forme galénique poreuse pour usage oral, comprenant un médicament, une matrice en PCL et d'autres adjuvants tels que notamment les agents lubrifiants et d'écoulement, est caractérisé en ce que, successivement:

a) on procède à la granulation du mélange comprenant la PCL ayant un poids moléculaire moyen de 2000 à 70 000, le médicament, l'agent lubrifiant, l'agent d'écoulement et le cas échéant d'autres adjuvants, pour obtenir une poudre dont les grains ont un diamètre compris entre 50 et 500 µm.

b) on procède à une compression de l'ensemble des composants de la préparation médicamenteuse sous une pression de 100 daN/cm$^2$ à 2000 daN/cm$^2$, puis

c) le cas échéant, on procède à un traitement choisi parmi l'ensemble constitué par (i) le traitement thermique à une température comprise entre 45 et 70 °C, pendant une durée inférieure ou égale à 20 minutes et (ii) le traitement au moyen d'un champ UHF (ultra-hautes fréquences) de 1 MHz à 25 GHz, pendant une durée inférieure ou égale à 60 s, et notamment comprise entre 10 et 60 s.

Selon l'invention, on peut obtenir des préparations médicamenteuses sous forme de comprimés pouvant renfermer jusqu'à 80% en poids de médicament.

Ces comprimés comprennent, outre la PCL et le médicament, un ou plusieurs adjuvants classiques en galénique, notamment un agent lubrifiant (notamment un stéarate de sodium, magnésium ou potassium), un agent d'écoulement (notamment de la silice colloïdale), un agent aromatisant et/ou un agent colorant.

De façon avantageuse, on utilisera 0,1 à 2% en poids d'agent lubrifiant par rapport au poids total de la préparation médicamenteuse, et de préférence, 0,5% en poids dudit agent lubrifiant.

De même, on utilisera 0,1 à 2% en poids (de préférence 0,5% en poids) d'agent d'écoulement par rapport au poids total de la préparation médicamenteuse.

D'autres avantages et caractéristiques de l'invention seront mieux compris à la lecture qui va suivre d'exemples de préparation nullement limitatifs mais donnés à titre d'illustration.

Préparation I

On procède à la granulation d'un mélange ayant une granulométrie de 125–200 µm et comprenant

– 89% en poids de PCL ayant les caractéristiques suivantes:

formule: $\{(CH_2)_5\text{–}CO\text{–}O\}_n$

n: 350 (valeur moyenne)

température de ramollissement: 58 °C densité: 0,2

masse moléculaire: 40 000 (environ) granulométrie: 125 à 200 µm

degré de cristallinité: 86%

– 10% en poids de KCl,

– 0,5% en poids de stéarate de magnésium, et

– 0,5% en poids de silice colloïdale.

On réalise à partir de ce mélange des comprimés de 12 mm de diamètre et pesant 350 mg chacun (sur une machine à comprimer alternative KORSH) par compression à 300 daN/cm$^2$.

On soumet ensuite les comprimés ainsi obtenus à un traitement thermique en étuve à 68 °C en faisant varier la durée dudit traitement pour étudier l'influence de ladite durée sur la dureté des comprimés.

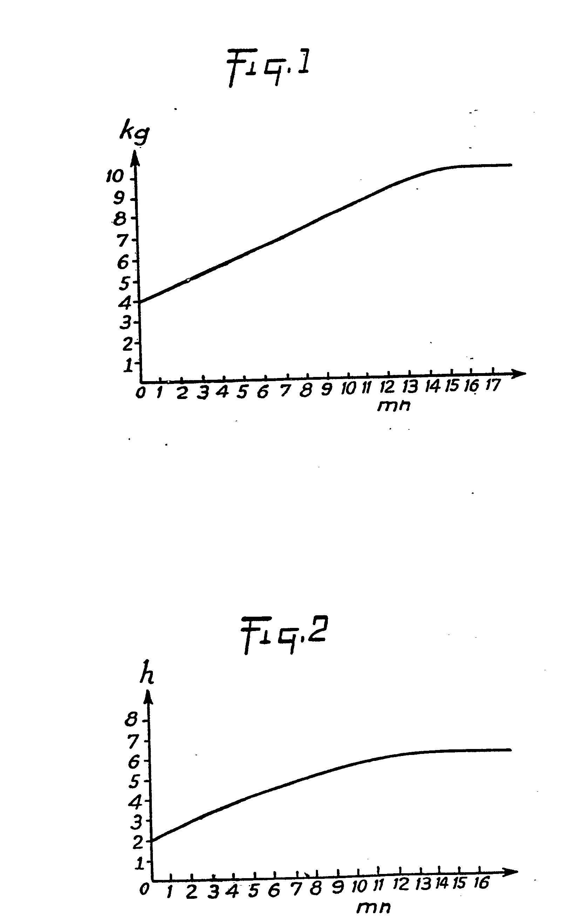

Les résultats obtenus ont permis de tracer la courbe de la figure 1 où la dureté (en ordonnée) exprimée en kg est donnée en fonction de la durée du traitement thermique (en abscisse) exprimée en minutes. On observe que (i) les matrices ne cassent pas, ni ne se brisent et (ii) dans le cas d'espèce un traitement thermique de 15 min suffit pour obtenir la dureté maximale.

Preparation II

On procède à la granulation sèche d'un mélange ayant une granulométrie de 125–200 µm et comprenant:

– 49% en poids de la PCL de la préparation I,

– 50% en poids de KCl,

– 0,5% en poids de stéarate de magnésium, et

– 0,5% en poids de silice colloïdale.

A partir du mélange ainsi granulé, on réalise des comprimés ayant un diamètre de 12 mm et pesant 350 mg par compression à 300 daN/cm$^2$.

Les comprimés obtenus sont soumis à un traitement thermique en étuve à 68 °C en faisant varier la durée dudit traitement pour étudier la cinétique de la libération du KCl.

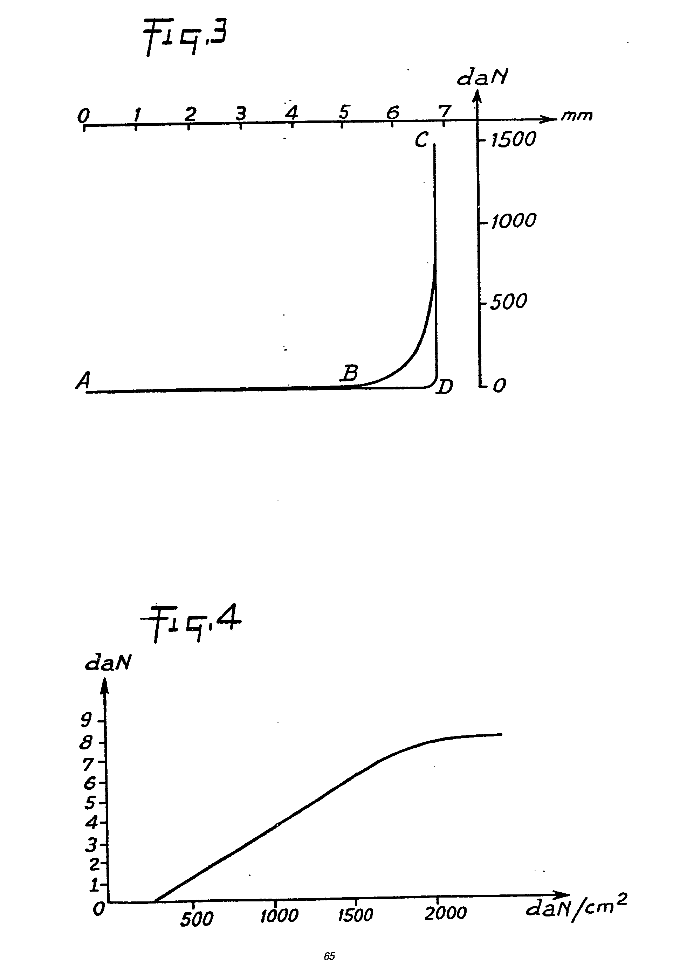

Les résultats obtenus ont permis de tracer la courbe de la figure 2 où la durée de libération de KCl (en ordonnée) exprimée en heutes est donnée en fonction de la durée du traitement thermique (en abscisse) exprimée en minutes. On constate que la durée de libération de KCl suit la loi de HIGUCHI et que la durée maximale de libération est atteinte après un traitement thermique de 14 min. Dans le cas d'espèce, le traitement thermique multiplie par 3 la libération.

Préparation III

A partir d'une poudre de PCL présentant les caractéristiques suivantes:
n: 300 (valeur moyenne)
densité: 0,2
masse moléculaire: 36 000 (environ)
granulométrie: 100 à 400 µm
degré de cristallinité: 93%
on a réalisé des comprimés (de 12 mm de diamètre et pesant 350 mg) renfermant KCl selon les modalités opératoires décrites dans la préparation II, le traitement thermique à 68 °C étant remplacé par un traitement UHF (fréquence: 2450 MHz; puissance 100 W), en faisant varier la durée dudit traitement UHF. L'influence de la durée de traitement sur la cinétique de la libération de KCl a été étudiée et les résultats correspondants consignés dans le tableau I.

Tableau I

Influence de la durée du traitement UHF sur la durée de libération de 50% en poids du KCl contenu dans les comprimés selon la préparation III

| Durée de traitement (en secondes) | Durée de libération de 50% du KCl (en heures) |
| --- | --- |
| 0 | 2 |
| 15 | 3 |
| 30 | 4 |
| 45 | 5 |
| 60 | 5 |

Lors de la préparation des comprimés selon la préparation III, on a étudié le cycle de compression et les résultats qui ont été obtenus ont permis de tracer la courbe de la figure 3. Ce cycle de compression représenté par le déplacement du poinçon supérieur (en abscisse) exprimé en mm et la force de compression (en ordonnée) exprimée en daN, présente une période de tassement AB, une période de compression BC et une période d'éjection CDA. Par rapport au cycle de compression des comprimés classiques, on constate que la période de tassement est très importante, et qu'il n'y a pas de grippage. On observe par ailleurs que les matrices des comprimés selon l'invention présentent une plasticité élevée et qu'elles conservent leurs caractéristiques géométriques alors que les comprimés classiques gonflent en général après éjection.

Les mêmes comprimés ont été encore utilisés pour étudier l'influence de la pression de compression sur la dureté de la matrice. Les résultats obtenus sont représentés par la courbe de la figure 4 où la dureté (en ordonnée) exprimée en DaN est donnée en fonction de la pression de compression (en abscisse) exprimée en daN/cm². On a constaté au cours de cette étude que les comprimés ne cassent pas mais se déforment seulement sous l'influence de la compression.

Préparation IV

On procède à la granulation sèche d'un mélange de granulométrie 50–500 µm renfermant:
– 10, 20 ou 40% en poids de verapamil,
– 0,5% en poids de stéarate de magnésium,
– 0,5% en poids de silice colloïdale, et
– 89, 79 ou 59% en poids de PCL
ayant les caractéristiques suivantes:
n: 400 (valeur moyenne)
température de ramollissement: 60 °C
densité: 0,2
masse moléculaire: 45 000 (environ)
granulométrie: 50–500 µm
degré de cristallinité: 91%
Avec le mélange ainsi granulé, on prépare (sur machine à comprimer alternative KORSH) des comprimés de 12 mm de diamètre et d'épaisseur comprise entre 3,2 et 3,5 mm par compression à 1500 daN/cm².

On a ensuite soumis les comprimés renfermant 40% en poids de verapamil à un traitement thermique en étuve à 60 °C pendant 10, 15 ou 20 min.

On a ensuite mesuré la durée ($T_{1/2}$) de libération de la moitié de la quantité de verapamil contenu dans chaque type de comprimés. Les résultats de cette étude cinétique ont été consignés dans le tableau II.

Tableau II

Etude cinétique de la libération de 50% en poids du verapamil contenu dans les comprimés selon la préparation IV

| Teneur en verapamil (% en poids) | Durée du traitement thermique (minutes) | Temps de libération de la moitié de la quantité de verapamil (minutes) |
| --- | --- | --- |
| 10% | 0 | 250 |
| 20% | 0 | 240 |
| 40% | 0 | 110 |
| 40% | 10 | 185 |
| 40% | 15 | 260 |
| 40% | 20 | 310 |

Les résultats du tableau II montrent comment selon l'invention on règle le relargage du principe actif dans le temps.

Préparation V

On prépare des comprimés renfermant 19% en poids de PCL,

80% en poids de fénofibrate,

0,5% en poids de stéarate de magnésium et

0,5% en poids de silice colloïdale

selon les modalités opératoires décrites dans la préparation IV en utilisant la PCL de ladite préparation IV.

L'étude cinétique de la libération du fénofibrate [nomenclature systématique: 2-[4-(4-chloro-benzoyl)-phénoxy]-2-méthylpropionate d'iso-propyle] montre que l'on peut contrôler in vivo la libération du principe actif dans le temps.

Préparation VI

Selon les modalités opératoires de la préparation III, on prépare des comprimés à libération contrôlée renfermant

    – 60% en poids d'acide 2-[4-(4-chloroben-zoyl)-phénoxy]-2-méthylpropionique,

    – 39% en poids de la PCL de la préparation III,

    – 0,5% en poids de stéarate de magnésium, et

    – 0,5% en poids de silice colloïdale.

Préparation VII

Selon les modalités opératoires de la préparation IV, on prépare des comprimés renfermant

    59% en poids d'acide 2-[4-(4-chloro-α-hy-droxybenzyl)-phénoxy]-2-méthylpropionique, 40% en poids de PCL,

    0,5% en poids de stéarate de magnésium, et

    0,5% en poids de silice colloïdale.

**Revendications**

1. Matrice inerte en polycaprolactone pour administration orale d'un médicament, ladite matrice qui a une porosité et une dureté réglables étant caractérisée en ce que la polycaprolactone qu'elle renferme a un poids moléculaire compris entre 2000 et 70 000 et est sous forme de grains agglomérés ayant un diamètre moyen compris entre 50 et 500 μm, et présente un degré de cristallinité d'au moins 75%.

2. Matrice selon la revendication 1, caractérisée en ce que la polycaprolactone qu'elle renferme a un poids moléculaire moyen compris entre 30 000 et 45 000.

3. Matrice selon l'une quelconque des revendications 1 à 2, caractérisée en ce que le diamètre moyen des pores constitués par les interstices présents entre les grains de polycaprolactone est compris entre 0,002 μm et 100 μm.

4. Matrice selon l'une quelconque des revendications 1 à 3, caractérisée en ce que la polycaprolactone présente un degré de cristallinité compris entre 80 et 95%.

5. Procédé de préparation d'une forme galénique poreuse destinée à être administrée par voie orale au moyen d'une matrice en polycaprolactone selon l'une quelconque des revendications 1 à 4, caractérisé en ce que:

a) son soumet à une granulation le mélange comprenant la polycaprolactone ayant un poids moléculaire moyen de 2000 à 70 000, le médicament, un agent lubrifiant et un agent d'écoulement;

b) on soumet le mélange résultant à une compression sous une pression de 100 à 2000 daN/cm², puis

c) le cas échéant, on soumet les comprimés résultants à un traitement choisi parmi l'ensemble constitué par (i) le traitement thermique à une température comprise entre 45 et 70 °C pendant une durée inférieure ou égale à 20 min, et (ii) le traitement au moyen d'un champ d'ultra-hautes fréquences de 1 MHz à 25 GHz, pendant une durée inférieure ou égale à 60 s et notamment comprise entre 10 et 60 s.

6. Procédé selon la revendication 5, caractérisé en ce que la teneur en médicament dans le mélange du stade a) est inférieure ou égale à 80% en poids par rappot au poids dudit mélange.

7. Procédé selon la revendication 5, caractérisé en ce que la teneur en agent lubrifiant dans le mélange du stade a) est comprise entre 0,1 et 2% en poids (de préférence 0,5% en poids) par rapport au poids dudit mélange.

8. Procédé selon la revendication 5, caractérisé en ce que la teneur en agent d'écoulement dans le mélange du stade a) est comprise entre 0,1 et 2% en poids (de préférence 0,5% en poids) par rapport au poids dudit mélange.

9. Forme galénique poreuse destinée à être administrée par voie orale, caractérisée en ce qu'elle comporte de la polycaprolactone, un médicament, un agent lubrifiant et un agent d'écoulement et qu'elle est obtenue selon l'une des revendications 5 à 8.

**Patentansprüche:**

1. Inerte Matrix aus Polycaprolacton zur oralen Verabreichung eines Medikaments, welche Matrix, die eine regulierbare Porosität und Härte aufweist, dadurch gekennzeichnet ist, dass das Polycaprolacton, das sie enthält, ein Molgewicht zwischen 2000 und 70 000 hat und in Form von agglomerierten Körnchen mit einem mittleren Durchmesser zwischen 50 und 500 μm vorliegt und einen Kristallinitätsgrad von mindestens 75% aufweist.

2. Matrix nach Anspruch 1, dadurch gekennzeichnet, dass das Polycaprolacton, das sie enthält, ein Molgewicht zwischen 30 000 und 45 000 aufweist.

3. Matrix nach einem der Ansprüche 1 bis 2, dadurch gekennzeichnet, dass der mittlere Durchmesser der von den zwischen den Polycaprolactonkörnchen vorhandenen Zwischenräumen gebildeten Poren zwischen 0,002 μm und 100 μm beträgt.

4. Matrix nach einem der Ansprüche 1 bis 3, dadurch gekennzeichnet, dass das vorhandene Polycaprolacton einen Kristallinitätsgrad zwischen 80 und 95% aufweist.

5. Verfahren zur Herstellung einer porösen galenischen Form zur Verabreichung auf oralem Weg mittels einer Matrix aus Polycaprolacton nach einem der Ansprüche 1 bis 4, dadurch gekennzeichnet, dass

a) man die Mischung, umfassend das Polycaprolacton mit einem mittleren Molgewicht von 2000 bis 70 000, das Medikament, ein Gleitmittel und ein Fliessmittel, einer Granulierung unterzieht;

b) man die resultierende Mischung einer Kompression unter einem Druck von 100 bis 2000 daN/cm² unterwirft, und dann

c) gegebenenfalls die resultierenden Tabletten einer Behandlung, ausgewählt aus der Gruppe bestehend aus (i) der Wärmebehandlung bei einer Temperatur zwischen 45 und 70 °C während einer Dauer von unter oder gleich 20 min und (ii) der Behandlung mit einem Ultrahochfrequenzfeld von 1 MHz bis 25 GHz während einer Dauer von unter oder gleich 60 s, insbesondere zwischen 10 und 60 s, unterzieht.

6. Verfahren nach Anspruch 5, dadurch gekennzeichnet, dass der Gehalt an Medikament in der Mischung der Stufe a) unter oder gleich 80 Gew.-%, bezogen auf das Gewicht der Mischung, ausmacht.

7. Verfahren nach Anspruch 5, dadurch gekennzeichnet, dass der Gehalt an Gleitmittel in der Mischung der Stufe a) zwischen 0,1 und 2 Gew.-% (vorzugsweise 0,5 Gew.-%), bezogen auf das Gewicht der Mischung, ausmacht.

8. Verfahren nach Anspruch 5, dadurch gekennzeichnet, dass der Gehalt an Fliessmittel in der Mischung der Stufe a) zwischen 0,1 und 2 Gew.-% (vorzugsweise 0,5 Gew.-%), bezogen auf das Gewicht der Mischung, ausmacht.

9. Poröse galenische Form zur Verabreichung auf oralem Weg, dadurch gekennzeichnet, dass sie Polycaprolacton, ein Medikament, ein Gleitmittel und ein Fliessmittel umfasst und dass sie nach einem der Ansprüche 5 bis 8 erhalten ist.

## Claims

1. An inert polycaprolactone matrix for the oral administration of a drug, said matrix, which has adjustable porosity and hardness, is characterized in that the polycaprolactone which it contains has a molecular weight of between about 2000 and 70 000, is in the form of agglomerated grains having an average diameter of between 50 and 500 µm and has a degree of crystallinity of at least 75%.

2. The matrix according to claim 1, characterized in that the polycaprolactone which it contains has an average molecular weight of between 30 000 and 45 000.

3. The matrix according to any one of claims 1 to 2, characterized in that the average diameter of the pores made up of the interstices present between the polycaprolactone grains is between 0.002 µm and 100 µm.

4. The matrix according to any one of claims 1 to 3, characterized in that the polycaprolactone has a degree of crystallinity of between 80 and 95%.

5. A method for the preparation of a porous galenic form for oral administration by means of a polycaprolactone matrix according to any one of claims 1 to 4, characterized in that it comprises:

a) granulating a mixture comprising the polycaprolactone having an average molecular weight of 2000 to 70 000, the drug, a lubricant and a flow promoter,

b) subjecting the resulting mixture to compression under a pressure of 100 to 2000 daN/cm², and then, if appropriate,

c) subjecting the resulting tablets to a treatment chosen from the group consisting of (i) heat treatment at a temperature of between 45 and 70 °C for a period less than or equal to 20 minutes, and (ii) treatment with an ultra-high frequency field of about 1 MHz to about 25 GHz for a period less than or equal to 60 seconds and especially of between 10 and 60 seconds.

6. The method according to claim 5, characterized in that the proportion of drug in the mixture of stage a) is less than or equal to 80% by weight relative to the weight of the said mixture.

7. The method according to claim 5, characterized in that the proportion of lubricant in the mixture of stage a) is between 0.1 and 2% by weight (preferably 0.5% by weight) relative to the weight of the said mixture.

8. The method according to claim 5, characterized in that the proportion of flow promoter in the mixture of stage a) is between 0.1 and 2% by weight (preferably 0.5% by weight) relative to the weight of the said mixture.

9. Porous galenic form designed to be administered by oral route, characterized in that it comprises polycaprolactone, a drug, a lubricant, and a flow promoter, and in that it is obtained according to any one of claims 5 to 8.

# Fig.1

# Fig.2

Fig.3

Fig.4